(19) Europäisches Patentamt
European Patent Office
Office européen des brevets

(11) Publication number: **0 131 285**
**B1**

(12) **EUROPEAN PATENT SPECIFICATION**

(45) Date of publication of patent specification: **04.11.87**

(51) Int. Cl.$^4$: **C 07 H 15/24,** A 61 K 31/70

(21) Application number: **84107938.7**

(22) Date of filing: **06.07.84**

# (54) Enamine derivatives of daunorubicin and adriamycine and pharmaceutical compositions containing them.

(30) Priority: **08.07.83 PL 242953**

(43) Date of publication of application:
**16.01.85 Bulletin 85/03**

(45) Publication of the grant of the patent:
**04.11.87 Bulletin 87/45**

(84) Designated Contracting States:
**DE FR GB IT SE**

(56) References cited:

**JOURNAL OF MEDICINAL CHEMISTRY, vol. 22, no. 7, 1979, pages 912-918, American Chemical Society, Washington, US; G.L. TONG et al.: "Adriamycin analogues. 3. Synthesis of N-alkylated anthracyclines with enhanced efficacy and reduced cardiotoxicity"**

**JOURNAL OF ANTIBIOTICS, vol. XXX, no. 5, 1977, pages 425-426; B.K. HAMILTON et al.: "Microbial n-acetylation of daunorubicin and daunorubicinol"**

(73) Proprietor: **Politechnika Gdanska**
**ul. Majakowskiego 11/12**
**Gdansk (PL)**

(72) Inventor: **Stefanska, Barbara, Dr.Eng.Chem.**
**ul Rodakoskiego 1E/17**
**Gdansk-Oliwa (PL)**
Inventor: **Falkowski, Leonard**
**ul Elblaska 69A/12**
**Gdansk (PL)**
Inventor: **Borowski, Edward, Prof.Dr. Eng.Chem.**
**ul. Chrzanowskiego 78/4**
**Gdansk-Wrzeszcz (PL)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 131 285 B1

**Description**

This invention relates to new enamine derivatives of daunorubicin and adriamycin with general formulae 1 and 2, wherein R denotes hydrogen atom or a hydroxyl group as well as to pharmaceutical compositions containing them.

Among antineoplasic antibiotics from the anthracycline group particularly valuable chemotherapeutics used in the treatment of cancer, mainly leukemia, are adriamycin and daunorubicin. However, their practical application is limited by the occurrence of harmful side effects, first of all, considerable cardiotoxicity.

The heretofore attempts to lower the cardiotoxicity of these antibiotics consisted in their chemical modification. To the most important of these modifications belong modifications leading to the production of the following derivatives: N,N-dibenzyl-daunorubicin (G. Tong et al, J. Med. Chem. 1972, *22*, 911), 5-Iminodaunorubicin (G. Tong et al, J. Med. Chem. 1979 *22*, 36) as well as qualemycin being a chelate of adriamycin with trivalent iron (M. Gosalves et al, Europ. J. Cancer, v. 14, 1185 (1978)).

The new enamine derivatives of daunorubicin and adriamycin of the present invention have the general formulae 1 and 2, wherein R denotes a hydrogen atom or a hydroxyl group. Included are also their acid addition salts.

Electron absorption spectra and infrared spectra indicate that in the aforementioned derivatives the structure of an anthracycline ring is maintained. Further structural evidence has been given by mass spectrometry obtained by the method of the field desorption, as well as elementary analysis of the obtained compounds.

Unexpectedly it has been shown and confirmed by the examinations of cardiac muscle of rats after the intraperitoneal administration of daunorubicin and N-(2-ethoxycarbonyl-1-methylvinyl)-daunorubicin in 0.2, 10 and 20 mg/kg doses that N-(2-ethoxycarbonyl-1-methylvinyl)-daunorubicin causes considerably smaller morphological and ultrastructural changes than daunorubicin.

Preparations from cardiac muscle stained with hematoxylin and eosine have been examined by a microscope using normal and polarized light. By means of both of the afore-mentioned methods it has been shown that daunorubicin in the three doses investigated leads to formation of numerous focuses of subsegmentary contractions causing disturbances of the heart rhythm. N-(2-ethoxycarbonyl-1-methylvinyl)-daunorubicin does not cause such changes giving the picture of cells of cardiac muscle identical to that obtained in the control animals, a regular and alternating system of isotropic and anisotropic fringes (D. Chibowski, Z. Siezieniewska, Z. Kleinrok, B. Chmielewska "Morphological and ultrastructural exponents of early cardiotoxicity in rats after the administration of rubidomycine and its derivatives", Patologia Polska, 1983).

New enamine derivatives of daunorubicin and adriamycin, as well as the method of production thereof are illustrated in the examples presented below.

Example 1

To a solution of 0.53 g of daunorubicin as the free base in 100 ml of methylene chloride 1 ml of ethyl acetylacetate is added with constant agitation and the whole mixture is agitated for 12 hours at room temperature in a nitrogen atmosphere. The reaction is checked by means of thin-layer chromatography on silica gel with the system toluene:acetone 8:1. After being evaporated to a small volume, the final product is precipitated by an addition of a 1:1 mixture of diethyl ether and hexane and crystallized from a mixture of acetone and diethyl ether. 0.4 g of N-(2-ethoxycarbonyl-1-methylvinyl)-daunorubicin is obtained which corresponds to 60 percent of the theoretical yield with a melting point of 151—154°C, with decomposition. M.S.F.D. m/s 639 ($M^+$, 80 percent of relative intensity), 640 ($M^+ + 1$, 100 percent of relative intensity), IR (KBr) $\nu$ = 1590, 1610, 1650, 1670, 1715, 1720 $cm^{-1}$ (chelated and free carbonyl groups).

Elementary analysis for formula $C_{33}H_{37}O_{12}N$

| | C | H | N |
|---|---|---|---|
| calculated (%) | 61.96 | 5.83 | 2.19 |
| found (%) | 61.74 | 5.98 | 2.01 |

Example 2

0.53 g of daunorubicin as the free base is dissolved in 100 ml of methylene chloride, 1 ml of acetylacetone is added and agitated for 12 hours at room temperature in a nitrogen atmosphere. The solution is concentrated and the product is precipitated with diethyl ether. In this way 0.5 g of N-(1-methyl-2-oxo-1-butenyl)-daunorubicin is obtained which corresponds to 80% of theoretical yield, with a melting point of 209—210°C with decomposition. M.S.F.D. m/s 609 ($M^+$ – 80% of relative intensity), 610 ($M^+ + 1$, 100% of relative intensity), IR (KBr) $\nu$ = 1585, 1620, 1710 $cm^{-1}$ (chelated and free carbonyl groups).

Elementary analysis for formula $C_{32}H_{35}O_{11}N$

| | C | H | N |
|---|---|---|---|
| calculated (%) | 63.06 | 5.79 | 2.30 |
| found (%) | 62.75 | 5.87 | 2.07 |

Example 3

0.057 g of adriamycin as the free base is suspended in 20 ml of methylene chloride, 0.1 ml of acetylacetone is added and agitated at room temperature for 12 hours in a nitrogen atmosphere. The final

product is separated in a similar way as in Example 2 and 0.04 g of N-(1-methyl-2-oxo-1-butenyl)-adriamycin is obtained corresponding to 60% of theoretical yield with a melting point of 210—213°C, with decomposition. M.S.F.D. m/s 625 ($M^+$, 100% of relative intensity).

Elementary analysis for formula $C_{32}H_{35}O_{12}N$

| | | | |
|---|---|---|---|
| calculated (%) | C 62.49 | H 5.74 | N 2.28 |
| found (%) | 62.10 | 5.76 | 2.18 |

Antitumor activity of enamine of daunorubicine derivatives are compared with the native antibiotic is illustrated in the table given below, wherein:

$ED_{50}$ is the concentration of the substance being investigated leading to 50% inhibition of the increase of cellular protein growth with regard to the control after 48 hours of incubation with the compound investigated.

T/C (%) ratio of medium life time of the mice treated to the control mice.

$LD_{50}$ dose causing death after 24 hours of half the number of animals which have been treated with the compound investigated intraperitoneally administered.

TABLE

| Compound tested | $ED_{50}$ (g/ml) | T/C (%) Dose (mg/kg) 200.000 | 100.000 | 50.000 | 25.000 | 12.500 | 6.250 | 3.130 | $LD_{50}$ (mg/kg) |
|---|---|---|---|---|---|---|---|---|---|
| Daunorubicin | 0.01 | | | 102 | 127 | 112 | 112 | 108 | 135 |
| N-(2-ethoxycarbonyl-1-methylvinyl)-daunorubicin | 0.05 | 102 | 144 | 132 (128)* | 115 (119)* | 103 | 103 | 94 | 500 |
| N-(1-methyl-2-oxo-1-butenyl)-daunorubicin | 0.05 | toxic | 147 | 118 (144)* | 125 (131)* | 119 | 110 | 131 | 500 |

* Results from a second series of experiments.

## Claims

1. Enamine derivatives of daunorubicin and adriamycin having the general formulae I and II

(I) 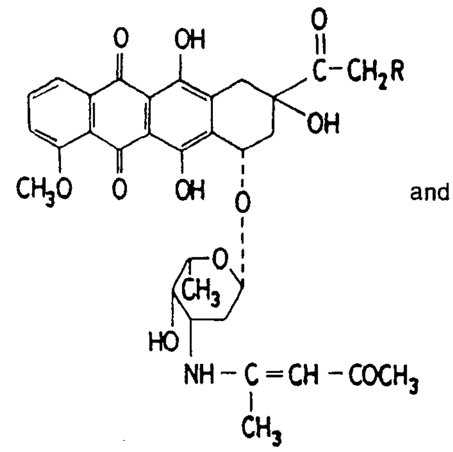 and 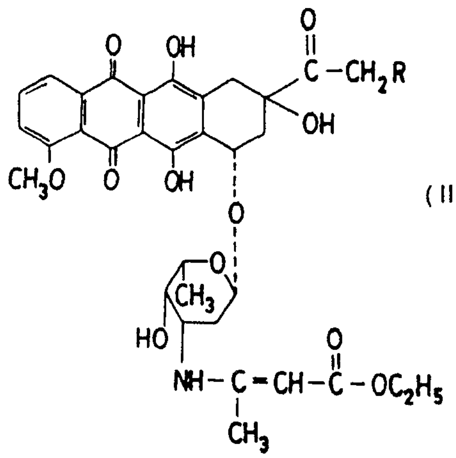 (II)

wherein R denotes a hydrogen atom or a hydroxyl group, as well as their acid addition salts.

2. Pharmaceutical compositions comprising at least one of the enamine derivatives of claim 1 or a pharmaceutically active acid addition salt thereof together with usual additives and carriers.

3. Pharmaceutical compositions according to claim 2 for use in treatment of cancer.

## Patentansprüche

1. Enamin-Derivate von Daunorubicin und Adriamycin der allgemeinen Formeln I und II

(I) 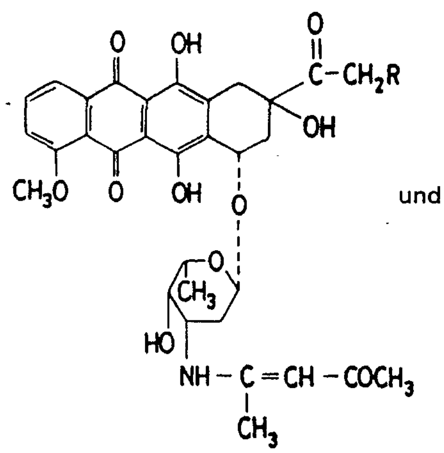 und 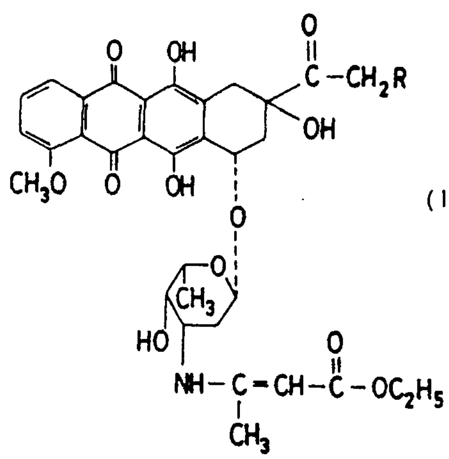 (II)

worin R ein Wasserstoffatom oder eine Hydroxylgruppe bedeutet, sowie deren Säureadditionssalze.

2. Pharmazeutische Zubereitungen, dadurch gekennzeichnet, daß diese mindestens ein Enaminderivat gemäß Anspruch 1 oder ein pharmazeutisch wirksames Säureadditionssalz desselben zusammen mit üblichen Zusatzstoffen und Trägern umfassen.

3. Pharmazeutische Zubereitungen nach Anspruch 2 zur Verwendung bei der Krebsbehandlung.

**Revendications**

1. Dérivés énamines de la daunorubicine et de l'adriamycine, présentant les formules générales I et II

(I) 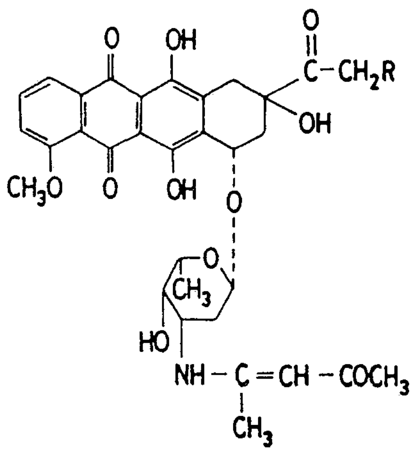

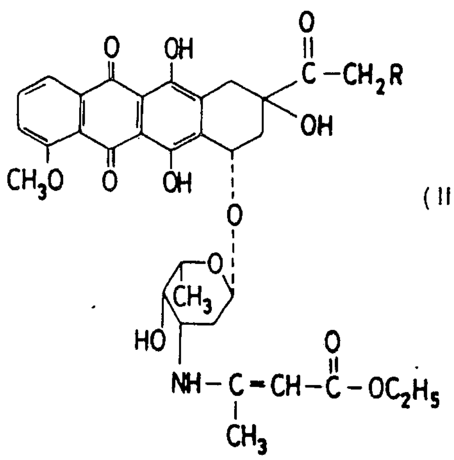 (II)

dans lesquelles R représente un atome d'hydrogène ou un groupe hydroxyle, ainsi que leurs sels d'addition d'acide.

2. Compositions pharmaceutiques comprenant au moins l'un des dérivés énamines de la revendication 1, ou un sel d'addition d'acide pharmaceutiquement actif de ceux-ci, conjointement avec des adjuvants et des véhicules habituels.

3. Compositions pharmaceutiques conformes à la revendication 2, à utiliser dans le traitement de cancers.

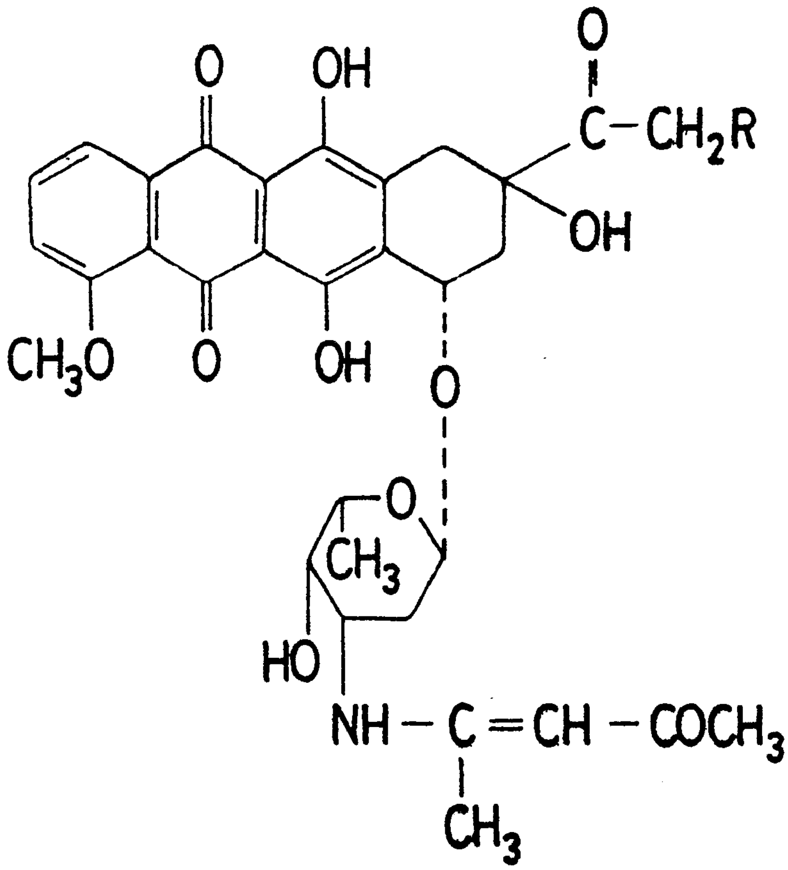

Form. 1

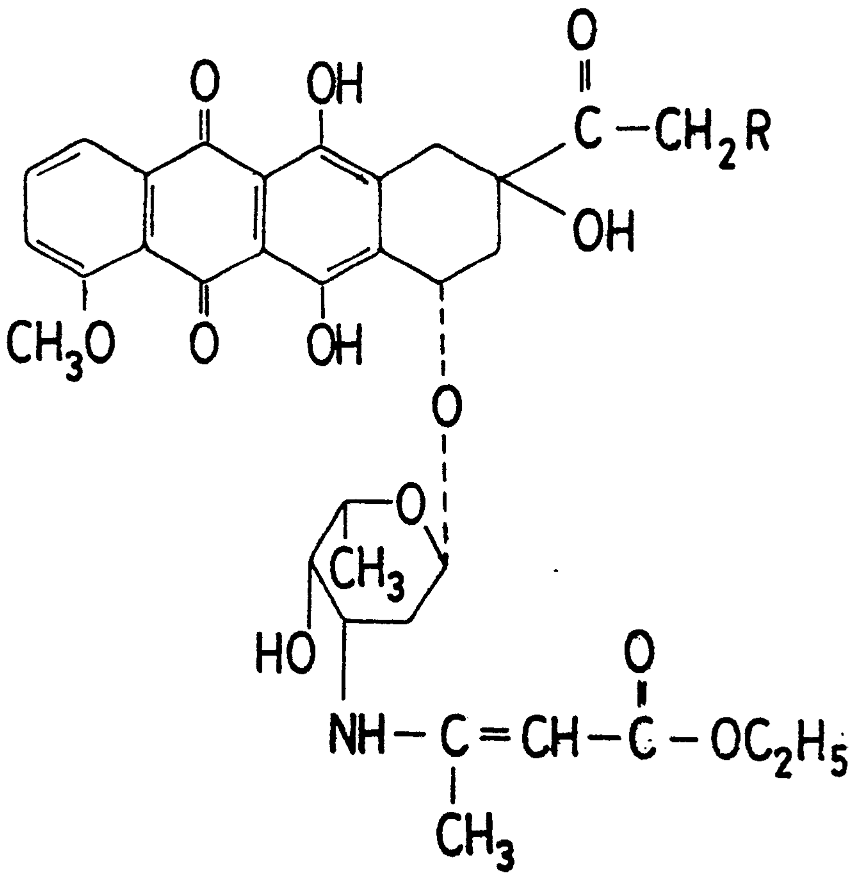

Form. 2